Europäisches Patentamt

European Patent Office

Office européen des brevets

(19)

(11) Publication number: **0 177 244 B1**

(12) **EUROPEAN PATENT SPECIFICATION**

(45) Date of publication of patent specification: 20.03.91

(51) Int. Cl.⁵: **C12Q 1/28**, G01N 33/72, G01N 33/52

(21) Application number: 85306766.8

(22) Date of filing: 24.09.85

(54) A method for the detection of peroxidase activity.

(30) Priority: 26.09.84 US 655471

(43) Date of publication of application:
09.04.86 Bulletin 86/15

(45) Publication of the grant of the patent:
20.03.91 Bulletin 91/12

(84) Designated Contracting States:
BE DE FR GB NL SE

(56) References cited:
EP-A- 0 030 684      EP-A- 0 085 261
EP-A- 0 121 317      GB-A- 779 921
GB-A- 2 030 292      US-A- 2 799 660

(73) Proprietor: WARNER-LAMBERT COMPANY
201 Tabor Road
Morris Plains New Jersey 07950(US)

(72) Inventor: Schumacher, Robert William
8 Corwin Street
Kenvil New Jersey 07847(US)
Inventor: Schobel, Alexander Mark
610 Somerset Street
North Plainfield New Jersey 07060(US)
Inventor: Moran, Richard George
41 New York Avenue
Lake Hopatcong New Jersey 07849(US)
Inventor: Rieger, Martin M.
304 Mountain Way
Morris Plains New Jersey 07950(US)
Inventor: Gallopo, Andrew Robert
133 Wessington Avenue
Garfield New Jersey 07026(US)

(74) Representative: Jones, Michael Raymond et al
HASELTINE LAKE & CO. Hazlitt House 28
Southampton Buildings Chancery Lane
London WC2A 1AT(GB)

EP 0 177 244 B1

## Description

This invention relates to a composition useful for the detection of peroxidase activity and in particular to the identification of peroxidase activity in various specimens. More particularly the invention relates to the preparation of a tableted indicator material which when admixed with a separate reagent solution, is useful for detection of peroxidase activity present in stool or biological fluids.

The detection of peroxidase activity has become an invaluable aid to the medical practitioner for the diagnosis of a number of disorders. One of the most widely used indicator reagents for diagnosing occult-blood is derived from an extract from the wood of certain species of trees of the Guaiacum genus native to the American tropics. The extract, termed guaiac, turns from essentially colorless to blue in the presence of hemoglobin and an oxidizing agent such as hydrogen peroxide. More specifically, the guaiac reagent is sensitive to what is termed "peroxidase activity" which results from the combination of an oxidizing agent with hemoglobin or certain chemically similar compounds.

Over 100,000 persons in the United States are affected by cancer of the colon and rectum per year, occurring equally in both the male and female population. When the number of colorectal cancers occurring each year is combined with the number of cancers occurring in other digestive organs, including the esophagus and stomach, such cancers of the digestive system account for more occurrences of cancer than any other single form of the disease. Contrary to may other forms of cancer, early diagnosis and treatment of digestive tract cancer does result in a cure rate of 80% to 90% of those persons affected by the disease. If, however, the disease is not detected until the later stages, the cure rate can drop drastically to 25% or less. Thus, early detection of the disease is critical to successful treatment of digestive tract cancer.

Most, but not all, cancers of the digestive tract bleed to a certain extent. Some blood found in the gastric contents and in vomitus is indicative of conditions associated with disorders of the mucous membrane, such as ulcers, diverticulitis, colitis, and carcinoma. In contrast, some blood is deposited on and in fecal matter excreted from the digestive system. The presence of blood in fecal matter is not normally detected, however, until gross bleeding occurs, resulting in blood visible to the naked eye. Gross bleeding, however, does not normally occur until the digestive tract cancers are in advanced stages.

It is known that digestive tract cancers in the early stages also tend to bleed, giving rise to occult (hidden) blood in fecal matter. Test equipment and test procedures have been developed for use by physicians in testing for the presence of occult blood in fecal matter. One of the most successful tests is manufactured and sold by SmithKline Diagnostics of Sunnyvale, California, under the trademark "Hemoccult."® The package for the "Hemoccult"® test is disclosed in U.S. Patent No. 3,996,006 issued to J. F. Pagano. Briefly, the Pagano test employs an absorbent white paper impregnated with a guaiac reagent encased in a special test slide having openable flaps on both sides of the test slide. To use the Pagano test slide, one must obtain a sample of fecal matter, smear it onto the guaiac-impregnated paper by opening the panel on one side of the test slide, and thereafter close the panel. A panel on the opposite side of the test slide is then opened and a developing agent, which is a stabilized solution of hydrogen peroxide and denatured alcohol, is applied to the guaiac-impregnated paper. If occult blood is present in the fecal matter smeared on the opposite side of the paper, the product of the guaiac reaction will appear as a blue substance against the white paper background, providing a positive indication of the presence of blood in the fecal matter.

Although the Pagano test is excellent for use by physicians in their offices and by diagnostic laboratories, it is not the type of test that is readily adaptable for use by the ordinary person because of his adverse reaction to handling fecal matter and because of his lack of skill in interpreting the results. As stated above, the Pagano test requires that a specimen of fecal matter be obtained. Normally, a specimen is obtained by procuring a sample on the end of a spatula or a wooden tongue depressor, which is then used to smear the specimen on the paper in the Pagano test slide. Once the sample is obtained and the test procedure completed, both the test slide and the spatula or depressor must be disposed of. Disposal of the used materials can and does present a physical problem to, if not an adverse psychological reaction for, the ordinary person. Thus, the ordinary person is not likely to use the Pagano test because of its uncleanly nature and because of the disposal problems associated with the used test slide and spatula or depressor. Additionally, the ordinary person does not necessarily have the skill required to analyze, and thus form accurate conclusions from, the test results.

As an alternative, the ordinary person can initiate the Pagano test in his home and then forward the test slide to his physician or a laboratory for addition of the developing agent and analysis of the test. This procedure, however, requires cold storage of the test slide and specimen if there is a significant time lapse before the test can be completed. Certainly, the ordinary person does not wish to store a fecal specimen in his household refrigerator, normally the only cold storage available to him, until he can present the specimen to his physician or an appropriate laboratory. Thus, the general public is not likely to follow or comply with this alternative.

Another test for occult blood is suggested by D. E. Fonner in the U.S. Patent No. 2,838,377. The Fonner test, as disclosed, can be effected in a toilet bowl containing fecal matter. The basic test reagents employed by Fonner are o-tolidine and benzidine. These reagents in the presence of blood and other reactants produce a dye visible to the naked eye. Although the Fonner test appears to be a solution to the problem of finding a viable home test for occult blood, it has not met with success for two reasons. First, the above-listed reagents are in themselves known to cause cancer and thus are not suitable for general public distribution. More importantly, the Fonner reagents have a relatively high rate of providing false indications of the presence of occult blood as a result of tap water impurities.

Nicholls and Fonner disclose in U.S. Patent No. 2,799,660 an occult blood test using a tablet composition containing a blood indicator, oxidizing agent, acetate compound and water-soluble solid acid. The tablet may additionally contain an effervescent agent. This test is being marketed by Ames under the trademark "Hematest." In determining the presence of absence of occult blood in a sample of urine a drop of the urine specimen is placed on a piece of dry filter paper, and when the drop has soaked into the paper a tablet prepared as above described is placed in the center of the drop and then two drops of water added to the tablet. With a positive test a ring of color appears on the filter paper surrounding the tablet, the color ranging from a very faint to a very deep blue, depending upon the concentration of blood in the sample.

To date, the use of the Pagano test, the Fonner test, and other similar tests has been limited primarily to physicians and diagnostic laboratories. Although this limitation might not at first glance present a soignificant problem, it does limit the early detection of digestive tract cancers, primarily because patients will not see a physician until other symptoms of digestive tract cancers, such as gross bleeding, manifest themselves. Thus, early detection of cancer of the digestive tract still does not occur with the great majority of patients who contract the disease. From EP-A-00885261 there is known a dry diagnostic aid comprising guaiac adsorbed on a carrier and an oxidizing agent. The diagnostic aid is for use "in bowl" and hence does not provide an accurate and reliable test for occult blood in faecal matter.

While the prior art has recognized the need for a simple and reliable test for detection of peroxidase activity, such test has not been available for routine home usage in an acceptable format.

A reagent system has been unexpectedly discovered which is useful for the in-home detection of peroxidase activity. The reagent system comprises a tablet containing indicator material which when admixed with an aqueous-organic reagent solution results in the formation of a stable, optically visible indicator for the presence of peroxidase activity, such as the presence of hemoglobin in a particular specimen or sample. The composition in the presence of peroxidase activity, such as blood, develops a visible color change. The reagent system and the tablet and reagent solution used to prepare it are stable at room temperature and are easily used in an acceptable manner rendering it effective for home usage. According to the present invention the test reagent system comprises (i) a tablet containing 3% to 40% gum guaiac, the remainder being excipients selected from disintegrating agents, lubricating agents, glidants, binders and diluents; and (ii) an aqueous-organic reagent solution containing an organic solvent for solubilizing the indicator, an oxidizing agent capable of oxidizing the indicator material in the presence of peroxidase activity, a buffer, and water.

The invention also contemplates a method for the detection of peroxidase activity and kit for use thereof which involves 1) collecting a specimen to be analyzed for the presence of peroxidase activity; 2) contacting the specimen, with a composition prepared by admixing a) a tablet containing about 3% to about 40% indicator material and remainder being an excipient selected from disintegrating agents, lubricating agents, glidants, binders and diluents; and b) an aqueous-organic reagent solution containing an organic solvent, an oxidizing agent being capable of oxidizing said indicator material in the presence of peroxidase activity, a buffer and water; and 3) detecting the presence or absence of peroxidase activity.

The instant inventive concept, like those of the prior art, are based on the detection of peroxidase activity present in stool and biological fluids. This peroxidase activity, also referred to as catalytically active substances, in the case of blood, has been associated with the presence of hemoglobin. These substances belong to the general class of hemoproteins and conjugate proteins all of which have the same prosthetic group, iron protoporphyrin or heme. This prosthetic group has the ability to catalyze the transfer of oxygen from an oxygen source to an acceptor which in turn becomes oxidized. The acceptor is a colorless precursor until it becomes oxidized wherein the oxidized form indicates the presence of the peroxidase activity by color formation.

The reagent system of this invention has enabled the preparation of an in-home diagnostic test for peroxidase activity, such as occult blood in stool using a tablet containing 3% to 40% gum guaic as indicator material, which when tableted is stable for long periods of time in the dry form and which, in combination with an aqueous-organic reagent solution, is likewise stable for long periods of time.

The instant invention represents a significant advance in the art by the preparation of a stable diagnostic reagent system which contains all of the essential materials necessary for the detection of peroxidase activity. This composition is used by merely contacting the specimen to be analyzed with the reagent system solution prepared by dissolving the tablet in the aqueous-organic reagent solution in one simple step and avoids the prior art manipulative handling of multiple reagents and specimens.

The tablet containing 3% to 40% gum guaic as indicator material is intended for use at the time a test is to be performed and results in the formation of a stable aqueous-organic reagent solution that is additionally stable for at least 9 months. The tableted indicator material in addition to being stable, must be capable of rapid dissolution in the aqueous-organic reagent solution. Rapidity of dissolution is essential to prepare a detection solution which is used within fifteen minutes after being admixed in order to result in consistent and uniform detection of occult blood without the formation of false negative results. In this regard, it has been found to be essential to prepare a tablet which contains the indicator material and high amounts of additional excipients.

The indicator material, gum guaiac, is capable of accepting oxygen and being oxidized to a colored dye in the presence of the oxygen source. As used herein, the term gum guaiac includes resin guaiac; individual components of resin guaiac such as alpha-guaiaconic acid, beta-guaiaconic acid, guaiacic acid and related compounds, guaiaretic acid and guaiacin; and mixtures thereof. This material is preferred because of its recognition as an effective indicator, its noncarcinogenicity and commercial availability. In the presence of an oxygen source and peroxidase activity, guaiac changes from colorless to a blue color.

The amount of indicator employed in the tablet of the invention is 3% to 40% gum guaic as dependent upon the sensitivity desired, that is the extent of color change evidenced from the oxidation reaction. Preferred useful amounts will vary on tablet size and texture but are consistent to yield from about 0.01% to about 3.0% and preferably about 0.25% to about 2.0% by weight, based upon the weight of the total reagent solution when dissolved. Amounts above about 3% are not desired since they may result in hypersensitivity showing false positive results. Amounts below about 0.01% are not recommended since they do not provide sufficient color development for accurate detection of peroxidase activity.

The tablets of the invention must be rapidly soluble in both water and the organic solvent used to prepare the reagent solution. The dimensions of the tablet are not critical but should be selected from suitable shapes that will facilitate rapid dissolution and convenience of handling to enable placement into the aqueous-organic reagent solution. The tablets hardness is selected from strengths that form a useable product that will not crumble during storage, that will not exhibit excessive friability and that will not resist erosion or dissolution when placed in the aqueous reagent solution. Suitable, nonlimiting hardness values, may range from about 3 to about 25 SC units (Strong-Cobb-Units) on edge using a Dr. K. Schleuniger & Company hardness tester (Model 2E) with preferred values up to 18 SC units. One SC unit equals 1 kilogram per $(2.54cm)^2$ (square inch).

When using the reagent system of the invention as a sprayable solution formulated with the gum guaiac tablet dissolved in the reagent solution, tablet weight should be such as to permit complete dissolution of the tablet into the reagent solution within minutes. Tablet weights of about 0.1 grams to about 1.5 grams and preferably 0.3 grams to about 0.75 grams have been found suitable for use in the invention.

As discussed above, tablets of the invention when prepared with gum guaiac as the indicator material must contain excipients to aid in stability, acceptable hardness levels and rapidity of dissolution. Gum guaiac is not compressible by conventional tabletting procedures and as such is incapable of being used alone. Even if tabletted, excessive dissolution times would result. Suitable tablets have been prepared which contain from about 3% to about 40% gum guaiac by weight of the total tablet and about 97% to about 60% by weight excipients of the total tablet.

The excipients used in the tablet are selected from disintegrating agents, lubricating agents, glidants, binders and diluents that are soluble in both water and the organic solvent present in the aqueous-organic reagent solution. Solubility in both materials is essential to prepare a pumpable, stable, optically clear indicator reagent system. In addition, the excipients must be nonreactive with the indicator materials and be compressible with the indicator to form a stable tableted reagent.

The disintegrants are designed to facilitate the rapid breakup of the tablet after it is admixed in the reagent solution. Disintegrants may be selected from a wide variety of agents and preferable are either swelling, wicking or effervescent types. Exemplary disintegrating agents, which normally are materials that tend to swell in the presence of water or draw in water and thus facilitate tablet wetting, include starch; starch derivatives such as low substituted carboxymethyl starches; clays such as Veegum HV and bentonite; celluloses such as purified cellulose, sodium carboxymethylcellulose and carboxymethylcellulose and cross-linked derivatives thereof as well as microcrystalline cellulose; and crosslinked polyvinylpyrrolidone. The amount of disintegrating agent may vary broadly and is preferably from about 1% to about 50% by weight of the tablet.

The effervescent disintegrants may be selected from a wide range of materials provided they are soluble in the reagent solution. Exemplary materials include an acid in solid dry form, such as citric, tartaric, succinic and so forth and a carbonate or bicarbonate which is preferably any alkaline earth or alkali metal carbonate or bicarbonate such as sodium and potassium. Calcium, barium and magnesium carbonates are not suitable since they are sparingly soluble in the aqueous-organic reagent solution and percarbonates and perborates are not fully acceptable since they are slowly soluble and/or reactive with the gum guaiac indicator once the final reagent system has been prepared. The amount of effervescent agent may range from about 5% to about 40% by weight of the tablet.

Lubricants are used in the tablet reagent in order to ease the ejection of the tablet from the die, to prevent sticking of the tablets to the punches and to limit wear on dies and punches. Lubricants may be selected from a wide range of materials which are both water and organic solvent soluble as well as finely divided particles such as calcium stearate, magnesium stearate, zinc stearate, stearic acid, hydrogenated vegetable oils, talc, light mineral oil, sodium benzoate, sodium lauryl sulfate, magnesium lauryl sulfate, polymeric fluorocarbons and mixtures thereof. The polymeric fluorocarbons may be selected from a well known group of polymeric and copolymeric substances made up of carbon and fluorine, which, in addition, may contain hydrogen and/or chlorine. The fluorocarbons may include at least one fluoroolefin; for example, polytetrafluoroethylene, and copolymers of tetrafluoroethylene and hexafluoropropylene are contemplated. Also included would be polyvinylidene fluoride, a copolymer of vinylidene fluoride and hexafluoropropylene, as well as other polymeric fluorocarbons recited in U.S. Pat. No 4,405,486 to Eoga. Polymeric fluorocarbons are the preferred lubricant in view of its ready availability and efficient lubrication properties.

The lubricants which are not jointly soluble should be in as fine a state of subdivision as possible to enable spraying through an orifice nozzle and because the smaller the particle size the greater the efficiency in the granulation or powder mix. Preferred sizes are those that pass through an 177 or 125 micrometre (80 or 100 mesh) screen (US Standard Mesh screen) and most preferred through a 74 micrometre (200 mesh)screen before use. The amount of lubricant will vary broadly and is preferably from about 0.1% to about 5% by weight of the total composition.

Glidants are used to improve the flow of granulations from hoppers into feed mechanisms and ultimately into the tabletting dye cavity. They often minimize the degree of surging occurring during direct compression and act to minimize the tendency of a granulation to separate or segregate due to excessive vibration. Glidants may be selected from a wide range of materials which are preferably water soluble or organic solvent soluble and if not jointly soluble should preferably be in as fine a state of subdivision as possible to enable spraying through an orifice nozzle. Preferred sizes are those that pass through an 177 or 125 micrometre (80 or 100 mesh) screen (US Standard Mesh size). Exemplary materials include talc, cornstarch, colloidal silicon dioxides and silicas.

Binders that are used when a wet granulation process is employed include starch, pregelatinized starch, gelatin, free polyvinylpyrrolidone, methylcellulose, sodium carboxymethylcellulose, polyvinylalcohols and so forth. Binders when used can be employed in amounts up to about 25% and preferably about 5% to about 15% by weight.

Diluents when used are selected to provide effective physicochemical changes to improve processing or manufacture as well as stability of tablet configuration. Diluents aid in providing a degree of cohesiveness when tablet size and rate of dissolution are important. Suitable diluents useful in the tablets of this invention include carbohydrates such as starch, dextrose and sucrose and polyhydric alcohols such as sorbitol, mannitol, xylitol, pentaerythritol and polymers thereof such as polyethers, nitrogen containing compounds such as urea and alkylureas, and mixtures thereof. These materials may be used in amounts of about 40% to about 97% by weight of the tablet.

The pressed tablets are prepared by conventional means using standard techniques and equipment known to those skilled in the art. Preferred procedures of preparing the tablets of this invention involves the direct compression method or wet granulation method.

In a typical direct compression method, the indicator composition in the form of a particulate solid is blended with the tablet formulation ingredients. Once incorporated, mixing is continued until a uniform mixture is obtained and thereafter the mixture is formed into suitable shapes by subjecting the formulation to a tabletting operation. Compression pressures on the order of up to 0.77 to $18.53 \times 10^7$ Pa (one-half to 12 tons per square inch) are normally employed.

In contrast, a typical wet granulation process for preparing a tablet involves milling of indicator and excipients, mixing of milled powders, preparation of binder solution, mixing of binder solution with powder

5

mixture to form wet mass, coarse screening of wet mass using 1680 micrometre (6 to 12 mesh) screen, drying of moist granules, screening of dry granules through a 1410 to 841 micrometre (14 to 20 mesh) screen, mixing of screened granules with lubricant and disintegrant and finally tablet compression. Variations hereof may be employed to insure compression of a viable tablet.

The reagent solution of the invention contains several essential ingredients: an organic solvent capable of solubilizing the indicator material, an oxidizing agent capable of oxidizing the indicator material in the presence of peroxidase activity, a buffer to maintain the pH value of the solution, and water. This solution should be separately storage stable for up to at least 24 months prior to admixing with the tabletted indicator material.

The organic solvent is present in the formulations of this invention to stabilize the oxidizing agent and to facilitate dissolution of the indicator material. A wide range of organic solvents may be used which are nonreactive with the formulation ingredients and which are preferably miscible in water.

Representative organic solvents include compounds selected from 1) alcohols, such as methanol, ethanol, propanol, isopropanol and isobutanol; 2) ketones, such as acetone, and mixtures thereof. Most preferred solvents are selected from the group consisting of methanol, ethanol, isopropanol and mixtures thereof.

The amount of solvent employed is that amount which is sufficient to keep gum guaiac in solution. This amount may vary widely and preferable ranges in amounts up to 75% by weight preferably 40-70% by weight of the total formulation. The exact amount of solvent for a particular formulation can be readily determined since it is that amount which is added to the remaining ingredients to yield a 100% solution.

The amount of water present in the solution will vary widely but must be sufficient to dissolve the oxidizing agent and buffer in order to maintain the pH value of the reagent solution. As such the aqueous-organic reagent solution may contain from about 5% to about 45% water and about 95% to about 55% organic solvent.

The ratio of the amount of water to organic solvent in the aqueous-organic reagent solution may be in the range of from 1:19 to 9:11 either by volume or weight.

The oxidizing agent is selected from hydrogen peroxide or materials that will yield hydrogen peroxide in the presence of water and are stable in the presence of the solvent. The hydrogen peroxide is the oxidizing agent that promotes the reaction between the indicator and the peroxidase activator to produce the indicator's color change. The oxidizing agent must be capable of reacting with the indicator only in the presence of the peroxidase activity containing material and remain stable in the formulation during storage.

Representative oxidizing agents include both organic and inorganic peroxides. Illustrative compounds include hydrogen peroxide, urea peroxide, potassium persulfate and mixtures thereof.

The oxidizing agent is employed in amounts of about 0.15% to about 3.0% by weight, based on the weight of formulation, and preferably from about 1.0% to 2.0% by weight. Higher amounts are not preferred since they may result in premature oxidation of the indicator after the indicator is added and possible precipitation of ingredients during storage whereas lower amounts will not enable sufficient oxidation for visual detection, possible slow reactivity and loss of peroxidase activity.

A buffer is employed in the formulation to aid in stability and to provide the optimum pH value to enable catalytic activity to occur. It is essential that the buffer selected maintain the pH within a range in which the indicator material changes color upon oxidation. Normally this will be between a pH of about 2 and about 8 and preferably between about 3.5 and about 6.5. Higher pH values should be avoided to prevent autoxidation of the indicator resulting in false positive results. Lower pH values do not result in efficient oxidation.

Representative buffers include citrate, tartrate, phosphate, acetate and mixtures thereof with citrate being preferred.

The aqueous-organic reagent solution of the invention may be prepared by routine procedures. The exact manner of mixing the reagents is not critical.

An alternate embodiment of the invention involves coating a previously prepared tablet with gum guaiac and a suitable film forming material. This embodiment enables an initial rapid dissolution of guaiac to permit immediate use of the reagent system while allowing additional time for tablet dissolution and subsequent use. Suitable film formers include hydroxypropyl methylcellulose, hydroxypropylcellulose, ethylcellulose, polyvinylpyrrolidone and mixtures thereof.

As will be readily recognized by one of ordinary skill, the present invention represents a significant advance of the prior art diagnostic aids employing reagent impregnated papers. All that need be done with the reagent system defined herein, is to dissolve the tablet containing the indicator material in a bottle containing the aqueous-organic reagent solution and then place the composition onto the contents to be tested for peroxidase activity and to observe the composition for the characteristic color change. The diagnostic aid and method for determining the presence of hemoglobin, such as in stool, through peroxidase activity in accordance with the present invention does not require the conventional handling of specimen, such as feces and can be simply performed in the home without need for professional interpretation of results.

In accordance with the present invention, a single container is employed containing all of the test reagents, which when admixed with the indicator and placed in contact with the specimen result in a characteristic color change resulting from the indicator oxidation reaction. This container is thus useful in a diagnostic kit for the detection of peroxidase activity.

The reagents may be dispensed in any convenient manner. Conventional dispensing means can include dropper bottle, spray delivery systems and aerosol delivery systems. Alternatively, conventional thickening additives may be employed in sufficient quantities to enhance thixotropy of the solution. Such solution can then be dispensed by roller means, dropper means and/or conventional physical manipulation. Such thixotropic additives may include but are not limited to silica gels, polyethylene glycol, methylcellulose, polyvinyl alcohol, poly(ethylene oxide) and quaternary ammonium derivatives.

The specimen may be obtained by routine collection procedures. Without being limited thereto, such procedures include standard techniques for isolating fecal occult blood, hemoglobin, biological fluids and so forth. The term biological fluids as used herein includes saliva, urine, gastric fluids, vaginal secretions and cervical secretions. Such specimens may be obtained using standard absorptive materials including paper strips and wood products. The actual method for collecting the specimen is considered to represent standard well-known technology readily available to those skilled in the art.

The following examples are given to illustrate the invention, but are not deemed to be limiting thereof. All percentages given throughout the specification are based upon weight unless otherwise indicated.

EXAMPLE I

This example demonstrated the preparation and use of an indicator tablet prepared by a wet granulation process.

A tablet having the formulation set forth in Table I was prepared by mixing the water with sodium bicarbonate and sodium carbonate until fully blended. The citric acid was then added and mixed for about 30 to 45 seconds. Thereafter gum guaiac was mixed until blended followed by the polytetrafluoroethylene powder.

The formulation was pressed using a Manesty®B3B Rotary Press with 0.79cm (5/16 inch) diameter FFBE (flat faced beveled edge tooling) tooling to yield a 0.1765 gram tablet having a 3 to 4 SC unit hardness on edge and dried for 1 hour at 60°C.

The tablets exhibited a good accelerated shelf stability for at least 3 months at 45°C, good ease of use and the capability to provide a positive test for hemoglobin within 5 minutes after immersion in an aqueous-organic reagent solution.

A peroxidase activity detection solution was prepared by dissolving one tablet in 15 ml of a reagent solution containing 5% w/v of a 30% hydrogen peroxide solution; 0.11% w/v citric acid (final solution pH 5.7-6.3); 0.25% w/v sodium citrate; 60% v/v of a methanol/ethanol solution (5 parts to 100 parts); 34.7% v/v water.

The effectiveness of this solution for detecting peroxidase activity was assessed by contacting previously prepared hemoglobin specimens with this solution. The hemoglobin specimens were prepared by taking diluted blood samples, drops of which were placed onto Whatman® No. 1 filter paper. A drop of the invention's formulation was applied to the diluted blood sample and a distinct change of color from colorless to blue was observed.

## TABLE I

| Ingredient | Pressed Tablet % w/w | Cured Tablet % w/w |
|---|---|---|
| Sodium bicarbonate | 22.7 | 22.9 |
| Sodium carbonate | 2.6 | 2.7 |
| Citric acid | 44.5 | 44.7 |
| Gum guaiac | 28.3 | 28.6 |
| Polytetrafluoroethylene powder | 1.1 | 1.1 |
| Water | 0.8 | 0 |

EXAMPLE II

This example demonstrates the preparation of an indicator tablet by a dry compression method.

A tablet having the formulation set forth in Table II was prepared by mixing the sorbitol and crosslinked polyvinyl pyrrolidone until blended. Thereafter the gum guaiac was blended followed by the remaining ingredients to prepare a dry homogenous mixture.

Tablets were prepared using 0.95cm (3/8 inch) FFBE tool to yield a tablet having a weight of 0.366 grams and a 6 to 8 SC unit hardness.

The procedure of Example I was employed to test the effectiveness of the tablet which dissolved in less than 4 minutes in the reagent solution and yielded a vivid blue color in contact with the diluted blood sample.

## TABLE II

| Ingredient | Amount % w/w |
|---|---|
| Crystalline sorbitol | 55 |
| Crosslinked polyvinyl pyrrolidone | 23 |
| Gum guaiac | 20 |
| Polytetrafluoroethylene powder | 1.8 |
| Silicon dioxide, colloidal | 0.2 |

EXAMPLE III

This example demonstrates the preparation of an indicator tablet without use of a disintegrant.

A tablet having the formulation of sorbitol, crystalline (82% w/w) gum guaiac (14% w/w) and polytetrafluoroethylene powder (4% w/w) was prepared by dry blending the first two ingredients followed by addition and blending of the third to prepare a homogenous mixture.

The formulation was pressed into tablets using a Stokes® DS 3 press and 1.27cm (1/2 inch) FFBE tooling to yield a tablet weighing 0.365 grams having a hardness of 12 to 15 SC units.

The procedure of Example I was employed to test the effectiveness of the tablets which dissolved in less than 15 minutes in the reagent solution and yielded a blue color upon contact with the diluted blood sample.

COMPARATIVE EXAMPLE IV

This example demonstrates the formation of gum guaiac tablets that are unsuitable for this invention.

A tablet having the formulation of sodium chloride (90.91% w/w), gum guaiac (6.99% w/w) and polytetrafluoroethylene powder (2.10% w/w) was prepared by dry blending the first two ingredients followed by addition and blending of the third to prepare a homogenous mixture.

The formulation was pressed into tablets using a Stokes DS 3 press and 1.27cm (1/2 inch) FFBE tooling to yield a tablet weighing 0.715 grams having a hardness of 5 to 10 SC units.

When the tablets were admixed with the reagent solution of Example I the comparative tablets produced instable solutions resulting from instability of gum guaiac with sodium chloride.

## Claims

1. A test reagent system for detecting the presence of peroxidase activity, which comprises:
   (i) a tablet containing from 3% to 40% of gum guaiac, the remainder being an excipient selected from disintegrating agents, lubricating agents, glidants, binders and diluents; and
   (ii) an aqueous-organic reagent solution containing water, an organic solvent, an oxidizing agent being capable of oxidizing the gum guaiac in the presence of peroxidase activity, and a buffer.

2. A test reagent system according to Claim 1 wherein the oxidizing agent is chosen from an organic peroxide, or an inorganic peroxide, preferably from hydrogen peroxide, urea peroxide, potassium persulphate and mixtures thereof.

3. A test reagent system according to Claim 1 or 2 wherein the buffer maintains the pH of the solution in the range of from 2.0 to 8.0, preferably 3.5 to 6.5.

4. A test reagent according to Claim 1, 2 or 3 wherein the organic solvent is chosen from alcohols, ketones, and mixtures thereof, preferably from methanol, ethanol, isopropanol and mixtures thereof.

5. A test reagent system according to Claim 1, 2, 3, or 4 wherein the aqueous-organic reagent solution contains :
   (a) in the range of from 0.15 to 3.0% by weight of the oxidizing agent;
   (b) an amount of buffer sufficient to maintain the pH value of the liquid solution in the range of 2.0 to 8.0;
   (c) an organic solvent capable of solubilizing the indicator material; and
   (d) water;
   all percentages being by weight of the final composition.

6. A test reagent system according to any preceding claim, wherein the aqueous-organic reagent solution contains from 5% to 45% water and at least 55% organic solvent.

7. A method for the detection of peroxidase activity, which method comprises the steps of :
   a) collecting a specimen which is to be analysed for the presence of peroxidase activity;
   b) contacting the specimen, with a composition prepared by admixing the tablet component (i) and the aqueous-organic reagent solution component (ii) of a test reagent system according to any one of claims 1 to 6.
   and

   c) detecting the presence or absence of peroxidase activity.

## Revendications

1. Un système de réactif de test de détection de présence d'une activité peroxydasique, qui comprend:
   (i) un cachet contenant de 3% à 40% de gomme guaiac, le reste étant composé d'un excipient choisi parmi les agents de désintégration, les agents de lubrification, les agents glissants, les liants et les diluants; et
   (ii) une solution aqueuse-organique de réactif contenant de l'eau, un solvant organique, un agent d'oxydation capable d'oxyder la gomme guaiac en présence d'une activité peroxydasique, et un tampon.

2. Un système de réactif de test selon la revendication 1, dans lequel l'agent d'oxydation est choisi parmi les peroxydes organiques ou inorganiques, et de préférence parmi le peroxyde d'hydrogène, le peroxyde d'urée, le persulfate de potassium et leurs mélanges.

3. Un système de réactif de test selon la revendication 1 ou 2, dans lequel le tampon maintient le pH de la solution à une valeur comprise entre 2,0 et 8,0, et de préférence entre 3,5 et 6,5.

4. Un système de réactif de test selon la revendication 1, 2 ou 3, dans lequel le solvant organique est choisi parmi les alcools, les cétones, et leurs mélanges, de préférence parmi le méthanol, l'éthanol, l'isopropanol et leurs mélanges.

5. Un système de réactif de test selon la revendication 1, 2, 3 ou 4, dans lequel la solution aqueuse-organique de réactif contient:
   (a) une proportion comprise entre 0,15% et 3,0% en poids d'agent d'oxydation;
   (b) une quantité de tampon suffisante pour maintenir le pH de la solution liquide à une valeur comprise entre 2,0 et 8,0;
   (c) un solvant organique capable de solubiliser le produit indicateur; et
   (d) de l'eau; tous les pourcentages étant exprimés par rapport au poids de la composition finale.

6. Un système de réactif de test selon l'une quelconque des revendications préférences, dans lequel la solution aqueuse-organique de réactif contient entre 5% et 45% d'eau, et au moins 55% de solvant organique.

7. Une méthode de détection d'une activité peroxydasique, laquelle méthode comprend les étapes de:
   (a) récupération d'un spécimen à analyser pour la présence d'activité peroxydasique;
   (b) mise en contact du spécimen avec une composition préparée par mélange du composant (i) cachet et du composant (ii) solution aqueuse-organique du réactif, composition qui forme le système réactif de test selon l'une quelconque des revendications 1 à 6; et
   (c) détection de la présence ou de l'absence d'activité peroxydasique.

## Ansprüche

1. Testreagenssystem zum Nachweis des Vorhandenseins einer Peroxidaseaktivität, umfassend:
   (i) eine Tablette mit 3 - 40% Guajakgummi und zum Rest einem Streckmittel, ausgewählt aus Sprengmitteln, Schmiermitteln, Gleitmitteln, Bindemitteln und Verdünnungsmitteln, und
   (ii) eine wäßrig-organische Reagenslösung mit Wasser, einem organischen Lösungsmittel, einem zur Oxidation des Guajakgummis bei Vorhandensein von Peroxidaseaktivität fähigen Oxidationsmittel und einem Puffer.

2. Testreagenssystem nach Anspruch 1, dadurch gekennzeichnet, daß das Oxidationsmittel aus einem organischen Peroxid, einem anorganischen Peroxid, vorzugsweise Wasserstoffperoxid, Harnstoffperoxid, Kaliumpersulfat und Mischungen derselben ausgewählt ist.

3. Testreagenssystem nach Anspruch 1 oder 2, dadurch gekennzeichnet, daß der Puffer den pH-Wert der Lösung im Bereich von 2,0 - 8,0, vorzugsweise 3,5 - 6,5, hält.

4. Testreagens nach Anspruch 1, 2 oder 3, dadurch gekennzeichnet, daß das organische Lösungsmittel aus Alkoholen, Ketonen und Mischungen derselben, vorzugsweise Methanol, Ethanol, Isopropanol und Mischungen derselben ausgewählt ist.

5. Testreagenssystem nach Anspruch 1, 2, 3 oder 4, dadurch gekennzeichnet, daß die wäßrig-organische Reagenslösung
   (a) 0,15 - 3,0 Gew.-% des Oxidationsmittels;
   (b) eine zur Aufrechterhaltung des pH-Werts der flüssigen Lösung im Bereich von 2,0 - 8,0 ausreichende Menge Puffer;
   (c) ein zum Inlösungbringen des Indikatormaterials fähiges organisches Lösungsmittel und
   (d) Wasser
   enthält, wobei sämtliche Prozentangaben sich auf das Gewicht der fertigen Masse beziehen.

6. Testreagenssystem nach einem der vorhergehenden Ansprüche, dadurch gekennzeichnet, daß die wäßrig-organische Reagenslösung 4 - 45% Wasser und mindestens 55% organisches Lösungsmittel enthält.

7. Verfahren zum Nachweis von Peroxidaseaktivität, umfassend folgende Stufen:
   (a) Sammeln einer auf das Vorhandensein von Peroxidaseaktivität hin zu analysierenden Probe;
   (b) Inberührungbringen der Probe mit einer durch Vermischen der Tablettenkomponente (i) und der wäßrigorganischen Reagenslösungskomponente (ii) eines Testreagenssystems nach einem der Ansprüche 1 bis 6 zubereiteten Masse und
   (c) Nachweis des Vorhandenseins oder Fehlens von Peroxidaseaktivität.